# EUROPÄISCHE PATENTSCHRIFT

(11) **EP 1 405 917 B1**
(45) Veröffentlichungstag und Bekanntmachung des Hinweises auf die Patenterteilung: **14.08.2013**
(21) Anmeldenummer: 03020847.4
(22) Anmeldetag: 13.09.2003
(51) Int. Cl.: C12P 17/00, C12P 41/00, C07D 213/55, C07D 213/56, C07D 333/24, C07D 333/60, C07D 307/54

(54) **Verfahren zur Herstellung von 3-Heteroaryl-3-hydroxy-propansäurederivaten durch enantioselektive mikrobielle Reduktion**
Process for the production of 3-Heteroaryl-3-hydroxy-propionic acid derivatives by enantioselective microbial reduction
Procédé de production de dérivés de l'acide 3-heteroaryl-3-hydroxy-propanoique par réduction énantiosélective microbienne

(30) Priorität: 26.09.2002 DE 10244811
(43) Veröffentlichungstag der Anmeldung: 07.04.2004
(73) Patentinhaber: Saltigo GmbH, 40764 Langenfeld (DE)
(72) Erfinder: Berendes, Frank, Dr., 48151 Münster (DE); Eckert, Markus, Dr., Shanghai 200050 (CN); Brinkmann, Nils, Dr., 51373 Leverkusen (DE); Dreisbach, Claus, Dr., 42799 Leichlingen (DE); Meissner, Ruth, Dr., 51375 Leverkusen (DE); Koch, Rainhard, Dr., 51065 Köln (DE)
(74) Vertreter: Wichmann, Birgid

(56) Entgegenhaltungen:
- EP-A- 1 153 919
- WO-A-03/078418
- WO-A1-98/54350
- FOGAGNOLO M ET AL: "Homochiral (R)- and (S)-1-heteroaryl- and 1-aryl-2-propanols via microbial redox" TETRAHEDRON: ASYMMETRY, ELSEVIER SCIENCE PUBLISHERS, AMSTERDAM, NL, Bd. 9, Nr. 13, 3. Juli 1998 (1998-07-03), Seiten 2317-2327, XP004131378 ISSN: 0957-4166
- FURUICHI A ET AL: "THE USE OF MICROORGANISMS IN ORGANIC SYNTHESIS 5. MICROBIOLOGICAL ASYMMETRIC REDUCTION OF METHYL-2-METHYL-3-2-THIENYL-3-OXO PROPIONATE" CHEMICAL AND PHARMACEUTICAL BULLETIN (TOKYO), Bd. 32, Nr. 4, 1984, Seiten 1619-1623, XP001179422 ISSN: 0009-2363
- ASHOK KUMAR ET AL: "A new chemoenzymatic enantioselective synthesis of R-(-)-tomoxetine, (R)- and (S)-Fluoxetine" TETRAHEDRON LETTERS, ELSEVIER SCIENCE PUBLISHERS, AMSTERDAM, NL, Bd. 32, Nr. 16, 1991, Seiten 1901-1904, XP002121769 ISSN: 0040-4039
- CHENEVERT R ET AL: "ASYMMETRIC SYNTHESIS OF BOTH ENANTIOMERS OF FLUOXETINE VIA MICROBIOLOGICAL REDUCTION OF ETHYL BENZOYLACETATE" TETRAHEDRON, Bd. 48, Nr. 33, 1992, Seiten 6769-6776, XP001179417 ISSN: 0040-4020
- QUIROS M ET AL: "Enantioselective reduction of beta-keto amides by the fungus Mortierella isabellina" TETRAHEDRON: ASYMMETRY, ELSEVIER SCIENCE PUBLISHERS, AMSTERDAM, NL, Bd. 8, Nr. 18, 25. September 1997 (1997-09-25), Seiten 3035-3038, XP004090508 ISSN: 0957-4166
- DEHLI J R ET AL: "Enantio- and chemoselective bioreduction of beta-keto nitriles by the fungus Curvularia lunata" TETRAHEDRON: ASYMMETRY, ELSEVIER SCIENCE PUBLISHERS, AMSTERDAM, NL, Bd. 11, Nr. 18, 22. September 2000 (2000-09-22), Seiten 3693-3700, XP004219718 ISSN: 0957-4166
- NAKAMURA KAORU ET AL: "Chiral synthesis of secondary alcohols using Geotrichum candidum", CHIRALITY, vol. 14, no. 9, 16 September 2002 (2002-09-16), pages 703-708, ISSN: 0899-0042

## Beschreibung

Die Erfindung betrifft ein Verfahren zur Herstellung von enantiomerenangereicherten 3-Heteroaryl-3-hydroxy-propansäurederivaten und 3-Heteroaryl-1-amino-propan-3-olen .

3-Heteroaryl-3-hydroxy-propansäurederivate und 3-Heteroaryl-1-amino-propan-3-ole haben insbesondere als Zwischenprodukte für die Herstellung von Arzneimitteln technische Bedeutung erlangt. So dienen einige 3-Heteroaryl-3-hydroxy-propansäurederivate und 3-Heteroaryl-1-amino-propan-3-ole beispielsweise als Vorläufersubstanzen für die Herstellung von Serotonin- oder Noradrenalin-Aufnahmeinhibitoren. Bei einigen dieser Inhibitoren konnte nachgewiesen werden, dass bestimmte Enantiomere nicht nur inaktiv oder weniger aktiv sind, sondern sogar unerwünschte Nebenwirkungen aufweisen können (US-A 5 104 899).

Ein Verfahren zur Herstellung von enantiomerenangereichertem (1S)-3-(Methylamino)-1-(2-thiophen-yl)-1-propanol ausgehend von 1-(2-Thiophen-yl)-3-chlorpropan-1-on wird in Chirality 2000, 12, 26-29 beschrieben. Nach der Reduktion zum racemischen 3-Chlor-1-(2-thienyl)-1-propanol wird das Racemat enzymatisch getrennt und das (S)-Enantiomer weiter mit NaI und Methylamin zu (S)-3-(Methylamino)-1-(2-thiophen-yl)-propan-1-ol umgesetzt. Diese Methode hat den Nachteil, dass bei enzymatischen Racemattrennungen prinzipiell nur 50 % des gewünschten Enantiomers erhalten werden können und die Gesamtausbeute daher wirtschaftlich nicht akzeptabel ist.

Es ist bereits bekannt, dass 3-Oxocarbonsäurederivate unter Verwendung von Mikroorganismen wie z. B. Hefen und Pilze enantioselektiv zu den entsprechenden enantiomerenangereicherten 3-Hydroxycarbonsäurederivaten reduziert werden können (siehe auch Sybesma et al., Biocatalysis and Biotransformation, 1998, Vol. 16, 95-134; Dahl et al., Tetrahedron: Asymmetry 10, 1999, 551-559, Dehli et al., Tetrahedron: Asymmetry 11, 2000, 3693-3700, Hayakawa et al, Tetrahedron Letters, 1998, Vol. 39, 67-70, Cabon et al., Tetrahedron: Asymmetry 6, 1995, 2199-2210 und Smallridge et al., Tetrahedron Letters, 1998, Vol. 39, 5121-5124).

Weiterhin ist in EP-A 447 938 ist die enantioselektive Synthese von 2-Halogeno-3-hydroxy-3-phenyl-propansäureestern durch Reduktion der 2-Halogeno-3-oxo-3-phenyl-propansäureester unter Verwendung verschiedener Mikroorganismen beschrieben.

Aus Chenevert et al., Tetrahedron 1992, Vol.48, 6769-6776 ist darüber hinaus die asymmetrische Synthese beider Enantiomere des Antidepressivums Fluoxetin (N-Methyl-3-(4-trifluormethylphenoxy)-3-phenylpropylamin Hydrochlorid) bekannt. Ein wichtiger Schritt der mehrstufigen Synthese ist die enantioselektive Reduktion des 3-Oxo-3-phenyl-propansäureethylesters mit Hilfe von Mikroorganismen.

Eine analoge Synthese ist in Kumar A. et al., Tetrahedron Letters, 1991, Vol. 32, 1901-1904 zur Herstellung des antidepressiv wirkenden (R)-Tomoxetine beschrieben. Auch hier ist die enantioselektive Reduktion des 3-Oxo-3-phenyl-propansäureethylesters zum 3-Hydroxy-3-phenyl-propansäureethylesters ein wichtiger Schritt.

Furuichi et al. , Chemical and Pharmaceutical Bulletin (Tokyo), Bd. 32, Nr. 4, 1984, S. 1619-1623 schreibt ein Verfahren zur Herstellung von Methyl 2-Methyl-3-(2-thienyl)-3-hydroxypropionat durch asymmetrische mikrobielle Reduktion von Methyl 2-Methyl-3-(2-thienyl)-3-oxopropionat, wie zum Beispiel mit Saccharomyces fermentati und Candida albicans. Aus dieser Literaturstelle ist zu entnehmen, dass die Reduktion eines Thienyl-β-Ketoesters mit Saccharomyces cereviseae nicht erfolgreich verläuft (siehe Seite 1620, Zeile 7-8).

Die enantioselektive Reduktion von Heteroarylketonen ist bislang jedoch nicht beschrieben worden.

Es bestand daher weiterhin das Bedürfnis, ein Verfahren bereitzustellen, das die Herstellung von enantiomerenangereicherten 3-Heteroaryl-3-hydroxy-propansäure-derivaten ermöglicht.

Es wurde nun ein Verfahren zur Herstellung von stereoisomerenangereicherten 3-Heteroaryl-3-hydroxypropansäurederivaten gefunden, das dadurch gekennzeichnet ist, dass

Verbindungen der Formel (VI),

Heteroaryl-CH(OH)-CH₂-CH₂-NR²R³ (VI)

in der

Heteroaryl für 2- oder 3-Thiophenyl, 2- oder 3-Furanyl oder 2-, 3-, oder 4-Pyridyl steht, wobei jeder der genannten Reste keinen, einen oder zwei Substituenten trägt, die jeweils unabhängig voneinander ausgewählt sind aus der Gruppe Methyl, Ethyl , n-Propyl, iso-Propyl, Cyano, Methoxy, Ethoxy, Fluor, Chlor, Trifluormethyl, Pentafluorethyl und Heptafluorisopropyl.
und
- R² und R³: jeweils unabhängig voneinander für Wasserstoff, C₁-C₈-Alkyl, C₄-C₁₄-Aryl oder C₅-C₁₅-Arylalkyl stehen oder die beiden Reste R² und R³ zusammen für C₃-C₁₂-Alkylen stehen, dadurch gekennzeichnet dass

- in einem Schritt a)
- Verbindungen der Formel (I)

   Heteroaryl-CO-CH₂W (I),

   in der Heteroaryl die vorstehend genannte Bedeutung besitzt,
   - W: für C(O)YR¹ₙ, wobei Y = für Sauerstoff steht und n = 1 ist oder Y für Stickstoff steht und n = 2 ist und
   - R¹: jeweils unabhängig voneinander für Wasserstoff, C₁-C₈-Alkyl, C₄-C₁₀-Aryl oder C₅-C₁₁-Arylalkyl oder im Falle, dass Y für Stickstoff steht, die beiden Reste R¹ zusammen für C₃-C₅ Alkylen stehen,
- in Gegenwart von Mikroorganismen in Form von Hefen und Pilzen ausgewählt aus der Gruppe *Saccharomyces* cereviseae, und/oder Zellpräparaten davon und
- in Gegenwart von Wasser mit einem pH-Bereich von 3 bis 11 bezogen auf 25°C
   umgesetzt werden und auf diese Weise enantiomerenangereicherte Verbindungen der Formel (II) erhalten werden,

   Heteroaryl-CH(OH)-CH₂W (II),

   in der Heteroaryl und W die vorstehend genannte Bedeutung besitzen und
- in einem Schritt b)
   i) für den Fall dass W für COOR¹ steht und R¹ für Wasserstoff, C₁-C₈-Alkyl, C₄-C₁₀-Aryl oder C₅-C₁₁-Arylalkyl steht, die enantiomerenangereicherten Verbindungen der Formel (II) mit Aminen der Formel (III)

      HNR²R³ (III)

      in der R² und R³ die unter der Formel (VI) genannte Bedeutung besitzen
      zu enantiomerenangereicherten Verbindungen der Formel (IV) umgesetzt werden

      Heteroaryl-CH(OH)-CH₂-CO-NR²R³ (IV)

      in der Heteroaryl, R² und R³ die vorstehend genannten Bedeutungen besitzen oder
   ii) für den Fall dass W für CON(R¹)₂ steht und die Reste R¹ jeweils unabhängig voneinander für Wasserstoff, C₁-C₈-Alkyl, C₄-C₁₀-Aryl oder C₅-C₁₁-Arylalkyl oder die beiden Reste R¹ zusammen für C₃-C₅ Alkylen stehen,
      die enantiomerenangereicherten Verbindungen der Formel (II) gegebenenfalls durch Umsetzung mit Aminen der Formel (III) zu enantiomerenangereicherten Verbindungen Formel (IV) umgesetzt werden und
- in einem Schritt c)
   die enantiomerenangereicherten Verbindungen der Formel (IV) durch Reduktion in enantiomerenangereicherte Verbindungen der Formel (VI) mit der oben genannten Bedeutung überfährt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Der Begriff enantiomerenangereichert umfasst im Sinne der Erfindung insbesondere enantiomerenreine Verbindungen oder auch beliebige Mischungen von Enantiomeren, in denen ein Enantiomeres in einem größeren Anteil als das andere Enantiomere, bevorzugt in einem relativen Anteil von 60 % bis 100 Mol-%, besonders bevorzugt 80 bis 100 Mol-% und ganz besonders bevorzugt 90 bis 100 Mol-% vorliegt.

**Alkyl** steht im Rahmen der Erfindung jeweils unabhängig für einen geradkettigen oder cyclischen, unabhängig davon verzweigten oder unverzweigten Alkyl-Rest, der durch C₁-C₄-Alkoxy-Reste weiter substituiert sein kann. Gleiches gilt für den nicht aromatischen Teil eines Arylalkyl-Restes.

Beispielsweise steht im Rahmen der Erfindung C₁-C₄-Alkyl für Methyl, Ethyl, 2-Ethoxyethyl, n-Propyl, Isopropyl, n-Butyl und tert.-Butyl, C₁-C₈-Alkyl darüber hinaus beispielsweise für n-Pentyl, Cyclohexyl, n-Hexyl, n-Heptyl, n-Octyl oder iso-Octyl. **Fluoralkyl** bedeutet jeweils unabhängig einen geradkettigen, cyclischen, verzweigten oder unverzweigten Alkyl-Rest, der einfach, mehrfach oder vollständig durch Fluoratome substituiert ist.

Beispielsweise steht C₁-C₄-Fluoralkyl für Trifluormethyl, 2,2,2-Trifluorethyl, Pentafluorethyl, Nonafluorbutyl und Heptafluorisopropyl.

**Aryl** steht im Rahmen der Erfindung beispielsweise und bevorzugt für carbocyclische aromatische Reste oder heteroaromatische Reste, die keines, ein oder zwei im gesamten heteroaromatische Rest mindestens jedoch ein Heteroatom enthalten, das ausgewählt aus der Gruppe Stickstoff, Schwefel oder Sauerstoff.

Weiterhin können die carbocyclischen aromatischen Reste oder heteroaromatischen Reste mit einem oder zwei Substituenten pro Cyclus substituiert sein, die jeweils unabhängig voneinander beispielsweise und bevorzugt ausgewählt sind aus der Gruppe Hydroxy, C₁-C₄-Alkyl, Cyano, COOH, COOM, wobei M für ein Alkalimetallion oder ein halbes Äquivalent eines Erdalkalimetallions steht, COO-(C₁-C₄-Alkyl), O-(C₁-C₄-Alkyl), N(C₁-C₄-Alkyl)₂, NH-(C₁-C₄-Alkyl), Fluor, Chlor, Brom, C₁-C₄-Fluoralkyl, CONH₂ oder CONH-(C₁-C₄-Alkyl). Gleiches gilt für den Arylteil eines Arylalkyl-Restes.

In den Formeln (I) und (II) steht Heteroaryl für 2-Thiophen-yl.

Bevorzugt steht R¹ für CN oder COOR¹, wobei R¹ für Wasserstoff oder Methyl oder Ethyl steht.

Bevorzugte Verbindungen der Formel (I) sind Methyl-3-oxo-3-(2-thiophen-yl)propanoat, Ethyl-3-oxo-3-(2-thiophen-yl)propanoat, Methyl-3-oxo-3-(3-thiophen-yl)propanoat, Ethyl-3-oxo-3-(3-thiophen-yl)propanoat, Methyl-3-oxo-3-(2-furan-yl)propanoat, Ethyl-3-oxo-3-(2-furan-yl)propanoat, Methyl-3-oxo-3-(3-furan-yl)propanoat, Ethyl-3-oxo-3-(3-furan-yl)propanoat, Methyl-3-oxo-3-(2-pyridin-yl)propanoat, Ethyl-3-oxo-3-(2-pyridin-yl)propanoat, Methyl-3-oxo-3-(3-pyridin-yl)propanoat, Ethyl-3-oxo-3-(3-pyridin-yl)propanoat, Methyl-3-oxo-3-(4-pyridin-yl)propanoat, Ethyl-3-oxo-3-(4-pyridin-yl)propanoat, 3-Oxo-3-(2-thiophen-yl)propansäurenitril, 3-Oxo-3-(3-thiophen-yl)propansäurenitril, 3-Oxo-3-(2-furan-yl)propansäurenitril, 3-Oxo-3-(3-furan-yl)propansäurenitril, 3-Oxo-3-(2-pyridin-yl)propansäurenitril, 3-Oxo-3-(3-pyridin-yl)propansäurenitril, 3-Oxo-3-(4-pyridin-yl)propansäurenitril und 3-Oxo-3-(2-thiophen-yl)propansäure-N-methylamid.

Als Mikroorganismen werden bevorzugt Hefen oder Pilzen ausgewählt aus der Gruppe *Saccharomyces cereviseae* eingesetzt.

Unter Zellpräparaten sind zu verstehen: gereinigte oder ungereinigte lysierte Zellen, die entweder feucht oder getrocknet, zum Beispiel als Lyophilisate eingesetzt werden können.

Bevorzugt ist der Einsatz von Mikroorganismen.

In einer bevorzugten Ausführungsform werden die Mikroorganismen vor der Umsetzung der der Verbindungen der Formel (I) auf komplexen oder mineralischen Nährmedien unter Anwendung von für die Anzucht der jeweiligen Mikroorgansimen an sich üblichen Kulturverfahren wie z.B. Kultivierung im Schüttelkolben, Batch-Fermentationen, Fed-Batch-Fermentationen bzw. kontinuierliche Fermentationen bis zu einer optischen Dichte von 1 bis 800, bevorzugt von 5 bis 300, gemessen bei einer Wellenlänge von 600 nm (OD₆₀₀), angezogen und gegebenenfalls nach der Anzucht konzentriert.

Die Anzucht kann beispielsweise bei Temperaturen zwischen 10 und 60°C erfolgen, bevorzugt zwischen 20 und 40°C.

Weiterhin kann der pH-Wert bei der Anzucht beispielsweise zwischen pH 3 und pH 9 liegen, bevorzugt zwischen pH 4 und pH 8, besonders bevorzugt zwischen pH 5 und pH 7,5. Dabei sind pH-Werte im gesamten Rahmen der Erfindung jeweils auf 25°C bezogen.

Die Anzucht kann unter aeroben und anaeroben Bedingungen erfolgen, bevorzugt wird sie aerob durchgeführt.

Für die Umsetzung wird in einer bevorzugten Ausführungsform die Verbindung der Formel (I) zu den Mikroorganismen gegeben, die entweder im Anzuchtmedium oder gegebenenfalls nach vorheriger Sedimentation in einer isotonischen Lösung resuspendiert vorliegen.

Die isotonische Lösung kann dabei eine mineralische Salzlösung oder ebenfalls ein Nährmedium für Mikroorganismen sein.

Die Mischung kann beispielsweise und bevorzugt geschüttelt oder gerührt und gegebenenfalls belüftet werden.

Das erfindungsgemäße Verfahren kann in einem pH-Bereich zwischen pH 3 bis pH 11, bevorzugt zwischen pH 4 bis pH 10 und besonders bevorzugt zwischen pH 6 bis pH 8 durchgeführt werden.

Das erfindungsgemäße Verfahren wird weiterhin üblicherweise bei einer Temperatur von 10 bis 60°C, vorzugsweise von 18 bis 45°C durchgeführt.

Die Reaktionsdauer kann 10 min bis 96 Stunden betragen, bevorzugt 60 min bis 72 Stunden und besonders bevorzugt 2 bis 48 Stunden.

Das erfindungsgemäße Verfahren kann so durchgeführt werden, dass die Verbindungen der Formel (I) einmalig, mehrmalig oder kontinuierlich zugegeben werden.

Die Summe der Konzentrationen der Verbindungen der Formeln (I) und (II) in der Zellsuspension kann zwischen 1 und 900 mM, bevorzugt zwischen 2 und 500 mM liegen, besonders bevorzugt zwischen 3 und 250 mM.

Zur Steigerung der Löslichkeit des Edukts im Reaktionsmedium können in einer bevorzugten Ausführungsform Hilfsstoffe wie polare mit Wasser mischbare Lösungsmittei wie zum Beispiel Glycerin, Dimethylformamid oder Dimethylsulfoxid oder andere Hilfsstoffe wie z.B. Cyclodextrine zugesetzt werden.

Weiterhin kann das erfindungsgemäße Verfahren in Gegenwart eines organischen Lösungsmittels z. B. in einem Mehrphasensystem wie insbesondere einem Zweiphasensystem durchgeführt werden.

Geeignete organische Lösungsmittel dafür sind beispielsweise mit Wasser nicht oder mit maximal 10 Vol.-% mischbare organische Lösungsmittel wie zum Beispiel aliphatische und aromatische gegebenenfalls chlorierte Lösungsmittel wie Petrolether, Hexan, Oktan, Heptan, Toluol, die isomeren Xylole, Chlorbenzol, Dichlormethan sowie Siliconöle. Oftmals kann auch das Edukt in Substanz als organische Phase eingesetzt werden.

Die Isolation der Verbindungen der Formel (II) können in an sich bekannter Weise beispielsweise durch Extraktion mit einem organischen Lösungsmittel, oder falls in einem Mehrphasensystem gearbeitet wurde, durch Abtrennung der organischen Phase und gegebenenfalls weiterer Extraktion und anschließendem Entfernen des organischen Lösungsmittels gewonnen werden.

Bevorzugt werden hierzu Lösungsmittel wie Toluol, Ethylacetat, Dichlormethan, Isobutylketon, Cyclohexan und Methylcyclohexan, bevorzugt Ethylacetat eingesetzt. Die Extraktion kann dabei durch kontinuierliche oder diskontinuierliche Zufuhr des Extraktionsmittels erfolgen. Im einfachsten Fall gelingt die Aufreinigung durch Ausschütteln mit den vorangehend erwähnten Extraktionsmitteln.

Falls erwünscht kann eine weitere Aufreinigung durch Destillation oder bei Raumtemperatur festen Verbindungen der Formel (I) durch Umkristallisation erfolgen.

Wird das erfindungsgemäße Verfahren in einem Mehrphasensystem durchgeführt, kann das Produkt auch direkt durch fraktionierte Destillation der organischen Phase isoliert werden.

Auf erfindungsgemäße Weise werden die enantiomerenangereicherten Verbindungen der Formel (II) gewonnen, wobei das stereogene Kohlenstoffatom, das die Heteroaryl- und die Hydroxygruppe trägt üblicherweise (S)-Konfiguration aufweist.

So eignet sich das erfindungsgemäße Verfahren zur Herstellung von (S)-Methyl-3-hydroxy-3-(2-thiophen-yl)propanoat, (S)-Ethyl-3-hydroxy-3-(2-thiophen-yl)propanoat, (S)-Methyl-3-hydroxy-3-(3-thiophen-yl)propanoat, (S)-Ethyl-3-hydroxy-3-(3-thiophen-yl)propanoat, (S)-Methyl-3-hydroxy-3-(2-furan-yl)propanoat, (S)-Ethyl-3-hydroxy-3-(2-furan-yl)propanoat, (S)-Methyl-3-hydroxy-3-(3-furanyl)propanoat,

(S)-Ethyl-3-hydroxy-3-(3-furan-yl)propanoat, (S)-Methyl-3-hydroxy-3-(2-pyridin-yl)propanoat, (S)-Ethyl-3-hydroxy-3-(2-pyridin-yl)propanoat, (S)-Methyl-3-hydroxy-3-(3-pyridin-yl)propanoat, (S)-Ethyl-3-hydroxy-3-(3-pyridin-yl)propanoat, (S)-Methyl-3-hydroxy-3-(4-pyridin-yl)propanoat, (S)-Ethyl-3-hydroxy-3-(4-pyridin-yl)propanoat, (S)-3-Hydroxy-3-(2-thiophen-yl)propansäurenitril, (S)-3-Hydroxy-3-(3-thiophen-yl)propansäurenitril, (S)-3-Hydroxy-3-(2-furan-yl)propansäurenitril, (S)-3-Hydroxy-3-(3-furan-yl)propansäurenitril, (S)-3-Hydroxy-3-(2-pyridin-yl)propansäurenitril, (S)-3-Hydroxy-3-(3-pyridin-yl)propansäurenitril, (S)-3-Hydroxy-3-(4-pyridin-yl)propansäurenitril und (S)-3-Hydroxy-3-(2-thiophen-yl)-propansäure-N-methylamid.

Bevorzugt stehen in den Formeln (III), (IV) und (VI) R² und R³ jeweils unabhängig für Wasserstoff, Methyl, Ethyl, Isopropyl, Phenyl oder Benzyl.

Besonders bevorzugt steht in den Formeln (III), (IV) und (VI) NR¹R² als Ganzes für Methylamino, Ethylamino und Isopropylamino.

Ganz besonders bevorzugt steht in den Formeln (III), (IV) und (VI) NR¹R² als Ganzes für Methylamino.

Die für das erfindungsgemäße Verfahren umfassend die Schritte a), b) und c) einsetzbaren Verbindungen der Formel (I) sind literaturbekannt oder analog zur Literatur herstellbar.

Vorzugsweise werden Verbindungen der Formel (I) in denen W nicht für CN steht durch Umsetzung von Verbindungen der Formel (VII)

Heteroaryl-CO-CH₃ (VII)

in der Heteroaryl die unter der Formel (I) genannte Bedeutung und Vorzugsbereiche besitzt
mit Verbindungen der Formel (VIII),

R¹-O-W (VIII)

in denen
- R¹ und W: die gleiche Bedeutungen besitzen, die unter der Formel (I), angegeben wurden, wobei W nicht für CN steht in Gegenwart einer Base gewonnen.

Beispielweise sei die Umsetzung von 2-Acetylthiophen mit Dimethylcarbonat, Diethylcarbonat, Diphenylcarbonat oder Dibenzylcarbonat, N-Methyl-methylcarbamat, N-Methyl-ethylcarbamat, N,N-Dimethyl-methylcarbamat oder N,N-Dimethyl-ethylcarbamat genannt.

Eine solche Umsetzung wird z.B. in Tetrahedron Lett. 1998, 39, 4995 beschrieben und kann beispielweise analog für die Umsetzung von 2-Acetylthiophen mit N-Methyl-methylcarbamat oder N-Methyl-ethylcarbamat zu 3-Oxo-3-(2-thiophen-yl)propansäure-N-methylamid angewandt werden.

Weiterhin können Verbindungen der Formel (Ia)

Heteroaryl-CH(OH)-CH₂-CO-NHR² (Ia)

Auch durch Umsetzung von Verbindungen der Formel (VII) mit Verbindungen der Formel (IX)

R²-NCO (IX)

in Gegenwart einer Base erhalten werden.

Im Schritt b) des erfindungsgemäßen Verfahrens werden die enantiomerenangereicherten Verbindungen der Formel (II) gemäß i), ii) oder iii) in an sich bekannter Weise in enantiomerenangereicherte Verbindungen der Formel (IV) überführt.

Eine Übersicht zur Darstellung von Carbonsäureamiden aus Carbonsäuren, Carbonsäureestem oder anderen Carbonsäureamiden beschreibt Houben Weyl "Methoden der Organischen Chemie", 4. Auflage, Band E 5, 941-1010.

Werden bei Raumtemperatur flüssige oder gasförmige Aminen der Formel (III) eingesetzt, ist die Verwendung von Lösungen der Amine bevorzugt. Beispielsweise können im Fall von Methylamin Lösungen aus Methylamin in Wasser, Methanol oder in Ethanol vorteilhaft für die Umsetzung von Verbindungen der Formel (II) in denen W für COOR¹ eingesetzt werden. Für die Überführung freier Carbonsäuren der Formel (II) in die Amide der Formel (IV) eignen sich beispielsweise Umsetzungen von Aminen der Formel (III) in Gegenwart von Kupplungsreagenzien wie 2-Halogenpyridinium oder 2-Halogen-1,3-thiazolium-Salzen oder in Gegenwart von sauren Kationenaustauschern.

Gemäß Schritt b) werden dann aus enantiomerenangereicherten Verbindungen der Formel (II) enantiomerenangereicherte Verbindungen der Formel (IV) erhalten.

Die Verbindungen der Formel (IV) können dann zu den Verbindungen der Formel (VI) reduziert werden. Die Reduktion von Carbonsäureamiden zu den entsprechenden Aminen ist prinzipiell bekannt und in Houben Weyl "Methoden der Organischen Chemie", 4. Auflage, Band E 16 d, 987-1003 zusammenfassend dargestellt. Bevorzugt ist die Umsetzung von Verbindungen der Formel (VI) mit komplexen Bor- oder Aluminiumhydriden wie z.B. Lithiumaluminiumhydrid, Red-Al^{®} (Natrium-bis-(2-methoxyethoxy)-dihydroaluminat oder Natriumborhydrid.

Besonders bevorzugt ist die Umsetzung von Verbindungen der Formel (VI) mit Lithiumaluminiumhydrid.

Die Reduktionen werden bevorzugt bei Temperaturen im Bereich Raumtemperatur bis 150°C durchgeführt, besonders bevorzugt im Bereich von 50 bis 110°C. Üblicherweise werden die Reduktionen in Ethern als Lösungsmittel durchgeführt, bevorzugt in cyclischen Ethern wie Tetrahydrofuran oder Dioxan, Reaktionen mit Red-Al^{®} lassen sich jedoch ebenso in Toluol als Lösungsmittel durchführen.

Auf erfindungsgemäße Weise erhält man die enantiomerenangereicherten Verbindungen der Formel (VI).

Als bevorzugte Verbindungen der Formel (VI) seien genannt:
(1S)-3-(Methylamino)-1-(2-thiophen-yl)-1-propanol, (1R)-3-(Methylamino)-1-(2-thiophen-yl)-1-propanol, (1S)-3-(Dimethylamino)-1-(2-thiophen-yl)-1-propanol, (1R)-3-(Dimethylamino)-1-(2-thiophen-yl)-1-propanol, wobei (1S)-3-(Methylamino)-1-(2-thiophen-yl)-1-propanol noch weiter bevorzugt ist.

Die erfindungsgemäß herstellbaren enantiomerenangereicherten Verbindungen der Formel (VI) eignen sich insbesondere zur Herstellung von enantiomerenangereicherten Verbindungen der Formel (X)

Heteroaryl-CH(OR⁶)-CH₂-CH₂NR²R³ (X)

in der
- Heteroaryl, R² und R³: die unter der Formel (I) angegebenen Bedeutungen und Vorzugsbereiche besitzen und
- R⁶: für Phenyl oder Naphthyl steht, das gar nicht, einfach oder mehrfach durch Substituenten substituiert sein kann, die jeweils unabhängig voneinander ausgewählt sind aus der Gruppe Cyano, CO-(C₁-C₁₂-Alkyl), O-(C₁-C₁₂-Alkyl), (C₁-C₁₂-Alkyl) Fluor, Chlor, Brom, C₁-C₁₂-Fluoralkyl.

Bevorzugt steht R⁶ für Naphthyl.

Bevorzugte Verbindungen der Formel (X) sind

(S)-N-methyl-3-(1-naphthalenyloxy)-3-(2-thienyl)propylamin und (R)-N-methyl-3-(1-naphthalenyloxy)-3-(2-thienyl)propylamin und deren Ammoniumsalze.

Vom Umfang der Erfindung ist daher auch ein Verfahren umfasst, das
- als Schritt d)
die Umsetzung von enantiomerenangereicherten Verbindungen der Formel (VI) mit Verbindungen der Formel (XI) zu enantiomerenangereicherten Verbindungen der Formel (X) in Gegenwart einer Base umfasst.

In Formel (XI)

R⁶-Hal (XI)

besitzt
- R⁶: die unter der Formel (X) genannte Bedeutung und

- Hal: steht für Fluor, Chlor, Brom oder Iod, bevorzugt für Fluor.

Als Verbindungen der Formel (XI) werden bevorzugt 1-Fluomaphthalin und 4-Chlorbenzotrifluorid eingesetzt.

Als Base können solche eingesetzt werden, die die Verbindungen der Formel (VI) an der Alkoholfunktion zumindest teilweise deprotonieren können.

Hierzu gehören beispielsweise Erdalkali- oder Alkalimetallhydride, -hydroxide, -amide, -alkoholate oder -carbonate wie beispielsweise Natriumhydrid, Natriumamid, Lithium-diethylamid, Natriummethylat, Natriumethylat, Kalium-tert.-butylat, Natriumhydroxid und Kaliumhydroxid.

Die erfindungsgemäß herstellbaren Verbindungen eignen sich insbesondere als Wirkstoffe in Arzneimitteln, wie insbesondere Serotonin- oder Noradrenalin-Aufnahmeinhibitoren, oder als Zwischenprodukte davon.

Das erfindungsgemäße Verfahren hat den Vorteil, dass ausgehend von einfach verfügbaren Edukten die Synthese von enantiomerenangereicherten 3-Heteroaryl-3-hydroxy-propansäurederivaten und 3-Heteroaryl-1-amino-propan-3-olen und deren Folgeprodukten in hohen Gesamtausbeuten, hohen Enantiomerenüberschüssen und hohen Reinheiten im technischen Maßstab möglich ist.

### Beispiele

### Beispiele 1 und 2:

### Reduktion von Methyl-3-oxo-(2-thiophen-yl)-propanoat durch verschiedene Stämme der Bäckerhefe Saccharomyces cereviseae.

Die Hefe-Stämme (*Saccharomyces cereviseae* NG 247, Uniferm GmbH & Co KG, Monheim; *Saccharomyces cereviseae* Y278, Deutsche Hefe Werke GmbH & Co oHG, Hamburg) wurden in mit 25 ml YM-Medium (Hefeextrakt, 3,0g/l; Malzextrakt, 3,0 g/l; Pepton 5,0 g/l; Dextrose, 10,0 g/l) befiillten 100-ml-Erlenmeyerkolben über Nacht bei 28°C unter Schütteln (200 rpm) angezogen.

Jeder der beiden Hefestämme wurden in drei 1-Liter-Erlenmeyerkolben, die mit 200 ml in YM-Medium gefüllt waren, bei 28°C unter Schütteln (200 rpm) inkubiert, die zuvor mit 12 ml Vorkultur beimpft worden waren. Das Wachstum wurde über die Messung der optischen Dichte bei 600 nm (OD₆₀₀) verfolgt. Nach 6-7 h Stunden erreichten die Kulturen eine OD₆₀₀ von 3 und wurden durch Zentrifugation geerntet (15 min, 8000 xg) und über Nacht bei 4°C im Kühlschrank gelagert. Für die Umsetzung wurden 5 ml des Zellpellets mit 250 µl 1 M Kaliumphosphat-Puffer (pH 7) und und 250 µl 1M Methyl-3-oxo-(2-thiophen-yl)-propanoat versetzt und in verschraubbaren 13-ml-Glasröhrchen geschüttelt. In regelmäßigen Abständen wurden 300 µl des Reaktionsansatzes entnommen und mit 300 µl Ethylacetat bzw. Toluol extrahiert. Nach anschließender Phasentrennungs-Zentrifugation (5 min, 5000 xg) wurde die organische Phase mittels chiraler Gaschromatographie analysiert. Die Ergebnisse sind in Tab. 1 aufgeführt.

**Tabelle 1**

| Reduktion von Methyl-3-oxo-(2-thiophen-yl)-propanoat durch verschiedene Hefe-Stämme. | | | | | | |
|---|---|---|---|---|---|---|
| **Beispiel** | **Hefe-Stamm** | **[c] Edukt** | **Reaktionszeit [h]** | **Produkt** | **Ausbeute** | **ee (S)** |
| 1 | NG 247 | 50 mM | 24 | 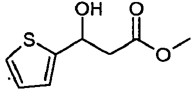 | 75 % | >97% |
| 2 | Y278 | 50 mM | 24 | 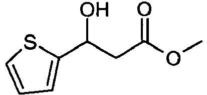 | 77 % | >97% |

### Beispiel 3:

### Reduktion von 3-(2-Thiophenyl)-3-oxo-propan-1-nitril durch Saccharomyces cereviseae Y278.

Die Anzucht der Hefezellen und die Umsetzung wurden wie in den Beispielen 1 und 2 beschrieben durchgeführt. Das Ergebnis ist in Tabelle 2 aufgeführt.

**Tabelle 2**

| **Beispiel** | **Hefe-Stamm** | **Edukt-konz.** | **Reaktionszeit [h]** | **Produkt** | **Ausbeute Produkt** | **ee (S)** |
|---|---|---|---|---|---|---|
| 3 | Y278 | 20 mM | 9 | 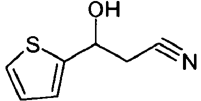 | 22 % | 85 % |

### Vergleichsbeispiele 4 und 5:

### Reduktion von 3-(2-Thiophenyl)-3-oxo-propansäuremethylesters durch Geotrichum candidum.

200 ml YM-Medium wurden in einem 1-Liter-Erlenmeyerkolben als 1. Vorkultur mit dem Stamm *Geotrichum candidum* ATCC34614 beimpft und 18 h bei 28°C unter Schütteln inkubiert. Als 2. Vorkultur wurden zwei 1-Liter-Erlenmeyerkolben mit je 200 ml GC-Medium (KH₂PO₄, 11,18 g/l; K₂HPO₄, 3,12 g/l; Glycerin, 30,0 g/l; Yeast Extract, 10,0 g/l; Polypeptone, 5,0 g/l) mit je 10 ml der 1. Vorkultur beimpft und ebenfalls 18 h bei 28°C geschüttelt.

Als Hauptkultur wurde ein 10-Liter-Fermenter mit 4,6 Liter GC-Medium beschickt und mit 400 ml der 2. Vorkultur beimpft. Die Anzucht erfolgte bei 28°C mit einer Belüftungsrate von 10 1/min und einer Rührgeschwindigkeit von 800 Upm. Nach 10 h wurde der Fermenter geerntet. Die Zellen wurden 15 Min. bei 6000 xg durch Zentrifugation sedimentiert, in wurden in 100 mM Kaliumphosphat-Puffer (KP-Puffer) pH 6,4 aufgenommen und im Kühlschrank bei 4°C gelagert.

16 g Feuchtbiomasse wurden mit 1,8g Glucose, 9 ml 1M KP-Puffer (pH 7,3) und 71 µl Endkonzentration 20 mM) bzw. 143 µl (Endkonzentration 40 mM) 3-(2-Thiophenyl)-3-oxo-propansäuremethylester versetzt und in einer 25-ml-Schottnasche mit einem Magnetrührer bei 28°C gerührt. In regelmäßigen Abständen wurden 300 µl des Reaktionsansatzes entnommen mit 300 µl Ethylacetat bzw. Toluol extrahiert und die organische Phase mittels Gaschromatographie analyisert. Die Ergebnisse sind in Tabelle 3 aufgeführt.

**Tabelle 3**

| Reduktion des 3-(2-Thiophenyl)-3-oxo-propansäuremethylesters durch den Stamm *Geotrichum candidum* ATCC34614. | | | | | |
|---|---|---|---|---|---|
| **Beispiel** | **Eduktkonz.** | **Reaktionszeit [h]** | **Produkt** | **Ausbeute Produkt** | **ee (S)** |
| 4 | 20 mM | 10 | 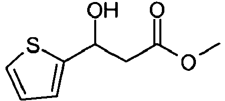 | 71 % | >98 % |
| 5 | 40 mM | 10 | 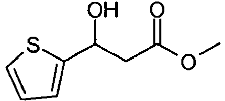 | 72 % | > 98 % |

### Beispiele 6 bis 12

### Reduktion verschiedener β-Ketoester durch Saccharomyces cereviseae.

Eine Vorkultur des Stamms *Saccharomyces cereviseae* NG247 wurde in 100 ml YM-Medium bei 28°C über Nacht unter Schütteln in einem 1-Liter-Erlenmeyerkolben angezogen. Als Hauptkultur wurden drei 1-Liter-Erlenmeyerkolben, die mit je 200 ml YM-Medium beschickt waren, mit je 10 ml der Vorkultur beimpft und bei 28°C geschüttelt. Nach 6 h hatten die Kulturen eine optische Dichte, die bei 600 nm gemessen wurde (OD₆₀₀), zwischen 7 und 8 erreicht. Die Zellen wurden durch Zentrifugation (15 min, 6000 xg) geerntet und als 10fache konzentriert Zellsuspension in 100 mM KP-Puffer (pH 7) + 3 %(w/v) Glucose resuspendiert. Von den Testsubstanzen wurden 1M ethanolischen Lösungen erstellt. In den Reaktionsansätzen wurden die Zellsuspension mit je 20 mM Testsubstanz versetzt und bei 30°C unter Schütteln inkubiert. In regelmäßigen Abständen wurden 300 µl des Reaktionsansatzes entnommen mit 300 µl Ethylacetat bzw. Toluol extrahiert und die organische Phase mittles Gaschromatographie analyisert. Die Ergebnisse sind in Tab. 4 aufgeführt.

**Tabelle 4**

| Enantioselektive Reduktion heterozyklischer β-Ketoester. | | | | | | |
|---|---|---|---|---|---|---|
| **Beispiel** | **Edukt** | **Edukt-konz.** | **Reaktionszeit** | **Produkt** | **Umsatz** | **ee** |
| 6 | 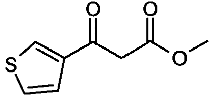 | 20 mM | 24 h | 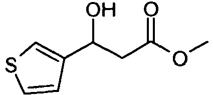 | 92 % | 98 % |
| 7 | 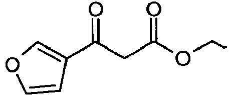 | 20 mM | 24 h | 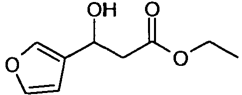 | 50 % | 87 % |
| 8 | 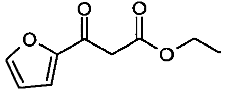 | 20 mM | 24 h | 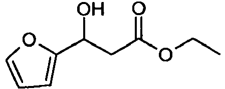 | 59 % | 93 % |
| 9 | 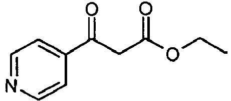 | 20 mM | 24 h | 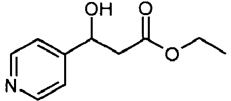 | 92 % | 81 % |
| 10 | 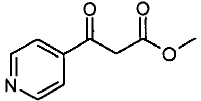 | 20 mM | 24 h | 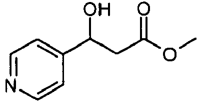 | 69 % | 96 % |
| 11 | 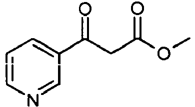 | 20 mM | 24 h | 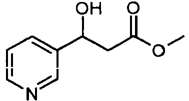 | 76 % | 96 % |
| 12 | 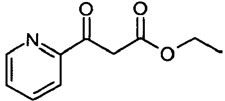 | 20 mM | 24 h | 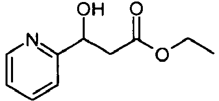 | 99 % | 99 % |

### Beispiel 13

### Herstellung von Methyl-3-oxo-(2-thiophenyl)-propanoat

In einen 2L-Kolben wurden 510 ml Dimethylcarbonat und 1500 ml Toluol auf 100°C erhitzt und anschließend innerhalb von 4 Stunden eine Lösung aus 257 g 2-Acetylthiophen in 510 ml Dimethylcarbonat zugetropft. Das in der Reaktion entstandene Methanol wurde dabei als Azeotrop abdestilliert. In einem 4 L Kolben wurden 120 ml konz. Schwefelsäure in 900 g Eis vorgelegt und das abgekühlte Reaktionsgemisch so zugegeben, dass 40°C nicht überschritten wurden. Es wurde nachgerührt und der pH-Wert auf pH 1 eingestellt. Die Phasen wurden getrennt und die organische Phase dreimal mit wässriger Natriumsulfatlösung ausgeschüttelt und anschließend im Vakuum eingeengt. Die Vakuumdestillation des Rohprodukts lieferte 278 g Methyl-3-oxo-(2-thiophenyl)-propanoat als transparente, leicht gelbliche Flüssigkeit (98 % rein nach GC, 74 % d. Th.).

### Beispiel 14

### Herstellung von N-Methyl-(3S)-3-hydroxy-3-(2-thienyl)propanamid

23 g Methyl (3S)-3-hydroxy-3-(2-thienyl)propanoat aus Versuchen gemäß den Beispielen 1 und wurden vorgelegt und mit 130 ml einer 2-molaren methanolischen Methylamin Lösung versetzt. Diese Mischung wird für 4 h bei 60°C gerührt, abgekühlt und anschließend im Vakuum eingeengt. So erhält man 24 g (Reinheit 87 %; 90 % d.Th.). Das Rohprodukt kann in so in die nächste Stufe eingesetzt werden oder aber aus Methylenchlorid und Hexan umkristallisiert werden. Dies lieferte 18 g N-Methyl-(3S)-3-hydroxy-3-(2-thienyl)propanamid (75 % d. Th.) in Form weißer Kristalle.

### Beispiel 15

### Herstellung von (1S)-3-(methylamino)-1-(2-thienyl)-1-propanol

350 ml trockenes Tetrahydrofuran werden mit 10 g Lithiumaluminiumhydrid vorgelegt und auf Rückfluss erhitzt. Gleichzeitig wird damit begonnen, 17 g N-Methyl-(3S)-3-hydroxy-3-(2-thienyl)propanamid aus Beispiel 14 gelöst in 150 g Tetrahydrofuran zuzutropfen. Nachdem alles zugetropft ist, wird über Nacht unter Rückfluss nachgerührt. Anschließend wird auf Raumtemperatur abgekühlt und es werden 200 ml Wasser vorsichtig zugetropft. Dann wurden 135 ml einer 10 %igen Natriumhydroxidlösung zugetropft und die Lösung filtriert. Das Lösungsmittel wurde im Vakuum entfernt. Die Rohlösung wurde mit 370 ml 1 N Natronlauge versetzt und 3 mal mit je 370 ml Toluol extrahiert. Die organischen Phasen werden vereinigt und die flüchtigen Bestandteile im Vakuum entfernt, so erhielt man 76 g (84 % Reinheit, 70 % d.Th.).

### Beispiel 16

### Reinigung von (1S)-3-(methylamino)-1-(2-thienyl)-1-propanol

15 g aus Beispiel 15 wurden in 150 ml Wasser in der Siedehitze gelöst und mit 5 g Aktivkohle versetzt und eine weitere Stunde unter Rückfluss nachgerührt. Die Suspension wurde heiß filtriert. Das Filtrat wurde dreimal mit je 100 ml Dichlormethan extrahiert. Die vereinigten organischen Phasen wurden eingeengt, der Rückstand in der Siedehitze in 50 ml Cyclohexan gelöst und während des Abkühlens mit 600 ml Petrolether kristallisiert. Die Kristalle wurden filtriert mit wenig Petrolether gewaschen und getrocknet. So erhielt man 12 g (1S)-3-(methylamino)-1-(2-thienyl)-1-propanol (98 % Reinheit, 93 % Ausbeute).

## Patentansprüche

1. Verfahren zur Herstellung von enantiomerenangereicherten Verbindungen der Formel (VI),
Heteroaryl-CH(OH)-CH₂-CH₂-NR²R³ (VI)
in der
Heteroaryl für 2- oder 3-Thiophenyl, 2- oder 3-Furanyl oder 2-, 3-, oder 4-Pyridyl steht, wobei jeder der genannten Reste keinen, einen oder zwei Substituenten trägt, die jeweils unabhängig voneinander ausgewählt sind aus der Gruppe Methyl, Ethyl , n-Propyl, iso-Propyl, Cyano, Methoxy, Ethoxy, Fluor, Chlor, Trifluormethyl, Pentafluorethyl und Heptafluorisopropyl.
und
R² und R³ jeweils unabhängig voneinander für Wasserstoff, C₁-C₈-Alkyl, C₄-C₁₄-Aryl oder C₅-C₁₅-Arylalkyl stehen oder die beiden Reste R² und R³ zusammen für C₃-C₁₂-Alkylen stehen, **dadurch gekennzeichnet dass**
• in einem Schritt a)
• Verbindungen der Formel (I)
Heteroaryl-CO-CH₂W (I),
in der Heteroaryl die vorstehend genannte Bedeutung besitzt,
W für C(O)YR¹ₙ, wobei Y = für Sauerstoff steht und n = 1 ist oder Y für Stickstoff steht und n = 2 ist und
R¹ jeweils unabhängig voneinander für Wasserstoff, C₁-C₈-Alkyl, C₄-C₁₀-Aryl oder C₅-C₁₁-Arylalkyl oder im Falle, dass Y für Stickstoff steht, die beiden Reste R¹ zusammen für C₃-C₅ Alkylen stehen,
• in Gegenwart von Mikroorganismen in Form von Hefen und Pilzen ausgewählt aus der Gruppe *Saccharomyces* cereviseae, und/oder Zellpräparaten davon und
• in Gegenwart von Wasser mit einem pH-Bereich von 3 bis 11 bezogen auf 25°C
umgesetzt werden und auf diese Weise enantiomerenangereicherte Verbindungen der Formel (II) erhalten werden,
Heteroaryl-CH(OH)-CH₂W (II),
in der Heteroaryl und W die vorstehend genannte Bedeutung besitzen und
• in einem Schritt b)
i) für den Fall dass W für COOR¹ steht und R¹ für Wasserstoff, C₁-C₈-Alkyl, C₄-C₁₀-Aryl oder C₅-C₁₁-Arylalkyl steht,
die enantiomerenangereicherten Verbindungen der Formel (II) mit Aminen der Formel (III)
HNR²R³ (III)
in der R² und R³ die unter der Formel (VI) genannte Bedeutung besitzen zu enantiomerenangereicherten Verbindungen der Formel (IV) umgesetzt werden
Heteroaryl-CH(OH)-CH₂-CO-NR²R³ (IV)
in der Heteroaryl, R² und R³ die vorstehend genannten Bedeutungen besitzen oder
ii) für den Fall dass W für CON(R¹)₂ steht und die Reste R¹ jeweils unabhängig voneinander für Wasserstoff, C₁-C₈-Alkyl, C₄-C₁₀-Aryl oder C₅-C₁₁ -Arylalkyl oder die beiden Reste R¹ zusammen für C₃-C₅ Alkylen stehen,
die enantiomerenangereicherten Verbindungen der Formel (II) gegebenenfalls durch Umsetzung mit Aminen der Formel (III) zu enantiomerenangereicherten Verbindungen Formel (IV) umgesetzt werden und
• in einem Schritt c)
die enantiomerenangereicherten Verbindungen der Formel (IV) durch Reduktion in enantiomerenangereicherte Verbindungen der Formel (VI) mit der oben genannten Bedeutung überführt werden.

2. Verfahren nach Anspruch 1, **dadurch gekennzeichnet, dass** in den Formeln (I) und (II) Heteroaryl für 2-Thiophen-yl steht.

3. Verfahren nach mindestens einem der Ansprüche 1 bis 2, **dadurch gekennzeichnet, dass** als Verbindungen der Formel (I) Methyl-3-oxo-3-(2-thiophen-yl)propanoat, Ethyl-3-oxo-3-(2-thiophen-yl)propanoat, Methyl-3-oxo-3-(3-thiophen-yl)propanoat, Ethyl-3-oxo-3-(3-thiophen-yl)propanoat, Methyl-3-oxo-3-(2-furan-yl)propanoat, Ethyl-3-oxo-3-(2-furan-yl)propanoat, Methyl-3-oxo-3-(3-furan-yl)propanoat, Ethyl-3-oxo-3-(3-furan-yl)propanoat, Methyl-3-oxo-3-(2-pyridin-yl)propanoat, Ethyl-3-oxo-3-(2-pyridin-yl)propanoat, Methyl-3-oxo-3-(3-pyridin-yl)propanoat, Ethyl-3-oxo-3-(3-pyridin-yl)propanoat, Methyl-3-oxo-3-(4-pyridin-yl)propanoat, Ethyl-3-oxo-3-(4-pyridin-yl)propanoat,1 oder 3-Oxo-3-(2-thiophen-yl)propansäure-N-methylamid eingesetzt werden.

4. Verfahren nach mindestens einem der Ansprüche 1 bis 3, **dadurch gekennzeichnet**, das die Summe der Konzentrationen der Verbindungen der Formeln (I) und (II) in der Zellsuspension zwischen 1 und 900 mM beträgt.

5. Verfahren nach mindestens einem der Ansprüche 1 bis 4, **dadurch gekennzeichnet, dass** das Verfahren in Gegenwart eines organischen Lösungsmittels durchgeführt wird.

6. Verfahren nach mindestens einem der Ansprüche 1 bis 5, **dadurch gekennzeichnet, dass** als Verbindungen der Formel (II) (S)-Methyl-3-hydroxy-3-(2-thiophen-yl)propanoat, (S)-Ethyl-3-hydroxy-3-(2-thiophen-yl)propanoat, (S)-Methyl-3-hydroxy-3-(3-thiophen-yl)propanoat, (S)-Ethyl-3-hydroxy-3-(3-thiophen-yl)propanoat, (S)-Methyl-3-hydroxy-3-(2-furan-yl)propanoat, (S)-Ethyl-3-hydroxy-3-(2-furanyl)propanoat, (S)-Methyl-3-hydroxy-3-(3-furan-yl)propanoat, (S)-Ethyl-3-hydroxy-3-(3-furan-yl)propanoat, (S)-Methyl-3-hydroxy-3-(2-pyridin-yl)-propanoat, (S)-Ethyl-3-hydroxy-3-(2-pyridin-yl)propanoat, (S)-Methyl-3-hydroxy-3-(3-pyridin-yl)propanoat, (S)-Ethyl-3-hydroxy-3-(3-pyridin-yl)propanoat, (S)-Methyl-3-hydroxy-3-(4-pyridin-yl)propanoat, (S)-Ethyl-3-hydroxy-3-(4-pyridin-yl)propanoat, oder (S)-3-Hydroxy-3-(2-thiophen-yl)-propansäure-N-methylamid hergestellt werden.

7. Verfahren nach mindestens einem derAnsprüche 1 bis 6, **dadurch gekennzeichnet, dass** in den Formeln (III), (IV) und (VI) R² und R³ jeweils unabhängig für Wasserstoff, Methyl, Ethyl, Isopropyl, Phenyl oder Benzyl stehen.

8. Verfahren nach mindestens einem der Ansprüche 1 bis 7, **dadurch gekennzeichnet, dass** Verbindungen der Formel (I) durch Umsetzung von Verbindungen der Formel (VII)
Heteroaryl-CO-CH₃ (VII)
in der Heteroaryl die unter der Formel (VI) genannte Bedeutung und Vorzugsbereiche besitzt mit Verbindungen der Formel (VIII)
R¹-O-W (VIII)
in denen
R¹ und W die gleiche Bedeutungen besitzen, die unter der Formel (I), angegeben wurden , in Gegenwart einer Base gewonnen werden

9. Verfahren nach einem oder mehreren Ansprüche 1 bis 8, **dadurch gekennzeichnet, dass** die Reduktion von Verbindungen der Formel (IV) mit komplexen Bor- oder Aluminiumhydriden erfolgt.

10. Verfahren nach mindestens einem der Ansprüche 1 bis 9, **dadurch gekennzeichnet, dass** (IS)-3-(Methylamino)-1-(2-thiophen-yl)-1-propanol, (1R)-3-(Methylaminoà)-1-(2-thiophen-yl)-1-propanol, (1S)-3-(Dimethylamino)-1-(2-thiophen-yl)-1-propanol oder (1R)-3-(Dimethylamino)-1-(2-thiophen-yl)-1-propanol hergestellt wird.

11. Verfahren nach mindestens einem der Ansprüche 1 bis 10, **dadurch gekennzeichnet, dass**
• in einem Schritt d)
die enantiomerenangereicherten Verbindungen der Formel (VI) in Gegenwart von Base mit Verbindungen der Formel (XI)
R⁶-Hal (XI)
in der
R⁶ für Phenyl oder Naphthyl steht, das gar nicht, einfach oder mehrfach durch Substituenten substituiert sein kann, die jeweils unabhängig voneinander ausgewählt sind aus der Gruppe Cyano, CO-(C₁-C₁₂-Alkyl), O-(C₁-C)₂-Alkyl]), (C₁-C₁₂-Alkyl) Fluor, Chlor, Brom, C₁-C₁₂-Fluoralkyl und
Hal für Fluor, Chlor, Brom oder Iod steht
zu enantiomerenangereicherten Verbindungen der Formel (X) umgesetzt werden,
Heteroaryl-CH(OR₆)-CH₂CH₂NR²R³ (X)
in den Heteroaryl, R² und R³ die unter der Formel (VI) und R⁶ die unter der Formel (XI) angegebene Bedeutung besitzt.

12. Verfahren nach Anspruch 11, **dadurch gekennzeichnet, dass** (S)-N-methyl-3-(1-naphthalenyloxy)-3-(2-thienyl)propylamin und (R)-N-methyl-3-(1-naphthalenyloxy)-3-(2-thienyl)propylamin oder deren Ammoniumsalze hergestellt werden.

## Claims

1. Process for preparing enantiomer-enriched compounds of the formula (VI),
heteroaryl-CH (OH) -CH₂-CH₂-NR²R³ (VI)
in which
heteroaryl is 2- or 3-thiophenyl, 2- or 3-furanyl or 2-, 3-, or 4-pyridyl, where each of the stated radicals contains no, one or two substituent(s) which is/are selected, in each case independently of each other, from the group methyl, ethyl, n-propyl, isopropyl, cyano, methoxy, ethoxy, fluorine, chlorine, trifluoromethyl, pentafluoroethyl and heptafluoroisopropyl,
and
R² and R³ are, in each case independently of each other, hydrogen, C₁-C₈-alkyl, C₄-C₁₄-aryl or C₅-C₁₅-arylalkyl, or the two radicals R² and R³ are together C₃-C₁₂-alkylene, **characterized in that**
• in a step a),
• compounds of the formula (I)
heteroaryl-CO-CH₂W (I),
in which heteroaryl has the above mentioned meaning,
W is C(O)YR¹ₙ, where Y is = oxygen and n is = 1 or Y is nitrogen and n is = 2, and
R¹ are, in each case independently of each other, hydrogen, C₁-C₈-alkyl, C₄-C₁₀-aryl or C₅-C₁₁-arylalkyl or, when Y is nitrogen, the two radicals R¹ are together C₃-C₅ alkylene,
• are reacted in the presence of microorganisms in the form of yeasts and fungi selected from the group *Saccharomyces* cereviseae, and/or cell preparations thereof, and
• in the presence of water having a pH range of
from 3 to 11, based on 25°C,
and, in this way, enantiomer-enriched compounds of the formula (II),
heteroaryl-CH(OH)-CH₂W (II),
in which heteroaryl and W have the abovementioned meaning, are obtained, and
• in a step b,)
i) when W is COOR¹ and R¹ is hydrogen, C₁-C₈-alkyl, C₄-C₁₀-aryl or C₅-C₁₁-arylalkyl,
the enantiomer-enriched compounds of the formula (II) are reacted with amines of the formula (III)
HNR²R³ (III)
in which R² and R³ have the meaning mentioned under formula (VI),
to give enantiomer-enriched compounds of the formula (IV)
heteroaryl-CH (OH) -CH₂-CO-NR²R³ (IV)
in which heteroaryl, R² and R³ have the above mentioned meanings, or
ii) when W is CON (R¹)₂ and the R¹ radicals are in each case, independently of each other, hydrogen, C₁-C₈-alkyl, C₄-C₁₀-aryl or C₅-C₁₁-arylalkyl, or the two R¹ radicals are together C₃-C₅-alkylene,
the enantiomer-enriched compounds of the formula (II) are converted, where appropriate by reacting with amines of the formula (III), into enantiomer-enriched compounds of the formula (IV), and
• in a step c),
the enantiomer-enriched compounds of the formula (IV) are converted, by means of reduction, into enantiomer-enriched compounds of the formula (VI) having the abovementioned meaning.

2. Process according to Claim 1, **characterized in that**, in the formulae (I) and (II), heteroaryl is 2-thiophenyl.

3. Process according to at least one of Claims 1 and 2, **characterized in that**, as compounds of the formula (I), use is made of methyl 3-oxo-3-(2-thiophenyl)propanoate, ethyl 3-oxo-3-(2-thiophenyl)propanoate, methyl 3-oxo-3-(3-thiophenyl)propanoate, ethyl 3-oxo-3-(3-thiophenyl)propanoate, methyl 3-oxo-3-(2-furanyl)propanoate, ethyl 3-oxo-3-(2-furanyl)propanoate, methyl 3-oxo-3-(3-furanyl)propanoate, ethyl 3-oxo-3-(3-furanyl)propanoate, methyl 3-oxo-3-(2-pyridinyl)propanoate, ethyl 3-oxo-3-(2-pyridinyl)propanoate, methyl 3-oxo-3-(3-pyridinyl)propanoate, ethyl 3-oxo-3-(3-pyridinyl)propanoate, methyl 3-oxo-3-(4-pyridinyl)propanoate, ethyl 3-oxo-3-(4-pyridinyl)propanoate and N-(methyl)-3-oxo-3-(2-thiophenyl)propanamide.

4. Process according to at least one of Claims 1 to 3, **characterized in that** the sum of the concentrations of the compounds of the formulae (I) and (II) in the cell suspension is between 1 and 900 mM.

5. Process according to at least one of Claims 1 to 4, **characterized in that** the process is carried out in the presence of an organic solvent.

6. Process according to at least one of Claims 1 to 5, **characterized in that**, as compounds of the formula (II), the following are prepared: methyl (S)-3-hydroxy-3-(2-thiophenyl)propanoate, ethyl (S)-3-hydroxy-3-(2-thiophenyl)propanoate, methyl (S)-3-hydroxy-3-(3-thiophenyl)propanoate, ethyl (S)-3-hydroxy-3-(3-thiophenyl)propanoate, methyl (S)-3-hydroxy-3-(2-furanyl)propanoate, ethyl (S)-3-hydroxy-3-(2-furanyl)propanoate, methyl (S)-3-hydroxy-3-(3-furanyl)propanoate, ethyl (S)-3-hydroxy-3-(3-furanyl)propanoate, methyl (S)-3-hydroxy-3-(2-pyridinyl)propanoate, ethyl (S)-3-hydroxy-3-(2-pyridinyl)propanoate, methyl (S)-3-hydroxy-3-(3-pyridinyl)propanoate, ethyl (S)-3-hydroxy-3-(3-pyridinyl)propanoate, methyl (S)-3-hydroxy-3-(4-pyridinyl)propanoate, ethyl (S)-3-hydroxy-3-(4-pyridinyl)propanoate, and N-(methyl)-(S)-3-hydroxy-3-(2-thiophenyl)propanamide.

7. Process according to at least one of Claims 1 to 6, **characterized in that**, in the formulae (III), (IV) and (VI), R² and R³ are, in each case independently, hydrogen, methyl, ethyl, isopropyl, phenyl or benzyl.

8. Process according to at least one of Claims 1 to 7, **characterized in that** compounds of the formula (I) are obtained by reacting compounds of the formula (VII)
heteroaryl-CO-CH₃ (VII)
in which heteroaryl has the meaning and preference ranges mentioned under formula (VI),
with compounds of the formula (VIII)
R¹-O-W (VIII)
in which
R¹ and W have the same meanings as those which were given under the formula (I), in the presence of a base.

9. Process according to one or more of Claims 1 to 8, **characterized in that** the reduction of compounds of the formula (IV) is effected using complex boron hydrides or aluminium hydrides.

10. Process according to at least one of Claims 1 to 9, **characterized in that** (1S) -3- (methylamino) -1- (2-thiophenyl)-1-propanol, (1R)-3-(methylamino)-1-(2-thiophenyl)-1-propanol, (1S)-3-(dimethylamino)-1-(2-thiophenyl)-1-propanol or (1R)-3-(dimethylamino)-1-(2-thiophenyl)-1-propanol is prepared.

11. Process according to at least one of Claims 1 to 10, **characterized in that**
• in a step d),
the enantiomer-enriched compounds of the formula (VI) are reacted, in the presence of base, with compounds of the formula (XI)
R⁶-Hal (XI)
in which
R⁶ is phenyl or naphthyl which can be substituted, not at all, once or more than once, by substituents which are selected, in each case independently of each other, from the group cyano, CO-(C₁-C₁₂-alkyl), O-(C₁-C₁₂-alkyl), (C₁-C₁₂-alkyl), fluorine, chlorine, bromine or C₁-C₁₂-fluoroalkyl, and
Hal is fluorine, chlorine, bromine or iodine,
to give enantiomer-enriched compounds of the formula (X),
heteroaryl-CH(OR⁶)-CH₂-CH₂NR²R³ (X)
in which heteroaryl, R² and R³ have the meaning given under formula (VI) and R⁶ has the meaning given under formula (XI) .

12. Process according to Claim 11, **characterized in that** (S) -N-methyl-3- (1-naphthalenyloxy) -3- (2-thienyl)propylamine and (R)-N-methyl-3-(1-naphthalenyloxy)-3-(2-thienyl)propylamine, or their ammonium salts, are prepared.

## Revendications

1. Procédé de fabrication de composés de formule (VI) enrichis énantiomériquement
hétéroaryle-CH(OH)-CH₂-CH₂-NR²R³ (VI)
dans laquelle
hétéroaryle représente 2- ou 3-thiophényle, 2- ou 3-furanyle ou 2-, 3- ou 4-pyridyle, chacun des radicaux cités ne portant aucun substituant ou portant un ou deux substituants, qui sont choisis à chaque fois indépendamment l'un de l'autre dans le groupe constitué par méthyle, éthyle, n-propyle, iso-propyle, cyano, méthoxy, éthoxy, fluor, chlore, trifluorométhyle, pentafluoroéthyle et heptafluoroisopropyle,
et
R² et R³ représentent chacun indépendamment l'un de l'autre hydrogène, alkyle en C₁-C₈, aryle en C₄-C₁₄ ou arylalkyle en C₅-C₁₅, ou les deux radicaux R² et R³ représentent ensemble alkylène en C₃-C₁₂, **caractérisé en ce que**
- lors d'une étape a)
- des composés de formule (I)
hétéroaryle-CO-CH₂W (I)
dans laquelle hétéroaryle a la signification indiquée précédemment,
W représente C(O)YR¹ₙ, avec Y = oxygène et n = 1 ou Y = azote et n = 2, et
les R¹ représentent chacun indépendamment l'un de l'autre hydrogène, alkyle en C₁-C₈, aryle en C₄-C₁₀ ou arylalkyle en C₅-C₁₁ ou, lorsque Y représente l'azote, les deux radicaux R¹ représentent ensemble alkylène en C₃-C₅,
- sont mis en réaction en présence de microorganismes sous la forme de levures et de champignons choisis dans le groupe *Saccharomyces* cereviseae et/ou leurs préparations cellulaires et
- en présence d'eau à un pH dans la plage allant de 3 à 11 à 25 °C,
et des composés de formule (II) enrichis énantiomériquement sont ainsi obtenus
hétéroaryle-CH (OH) -CH₂W (II)
dans laquelle hétéroaryle et W ont la signification indiquée précédemment, et
- lors d'une étape b)
i) lorsque W représente COOR¹ et R¹ représente hydrogène, alkyle en C₁-C₈, aryle en C₄-C₁₀ ou arylalkyle en C₅-C₁₁,
les composés de formule (II) enrichis énantiomériquement sont mis en réaction avec des amines de formule (III)
HNR²R³ (III)
dans laquelle R² et R³ ont la signification indiquée pour la formule (VI)
pour former des composés de formule (IV) enrichis énantiomériquement
hétéroaryle-CH (OH) -CH₂-CO-NR²R³ (IV)
dans laquelle hétéroaryle, R² et R³ ont les significations indiquées précédemment ou
ii) lorsque W représente CON(R¹)₂ et les radicaux R¹ représentent chacun indépendamment l'un de l'autre hydrogène, alkyle en C₁-C₈, aryle en C₄-C₁₀ ou arylalkyle en C₅-C₁₁ ou les deux radicaux R¹ représentent ensemble alkylène en C₃-C₅,
les composés de formule (II) enrichis énantiomériquement sont transformés en composés de formule (IV) enrichis énantiomériquement éventuellement par mise en réaction avec des amines de formule (III), et
- lors d'une étape c)
les composés de formule (IV) enrichis énantiomériquement sont transformés par réduction en des composés de formule (VI) enrichis énantiomériquement ayant la signification indiquée précédemment.

2. Procédé selon la revendication 1, **caractérisé en ce qu'**hétéroaryle représente 2-thiophényle dans les formules (I) et (II).

3. Procédé selon au moins l'une quelconque des revendications 1 à 2, **caractérisé en ce que** du propanoate de méthyl-3-oxo-3-(2-thiophényle), du propanoate d'éthyl-3-oxo-3-(2-thiophényle), du propanoate de méthyl-3-oxo-3-(3-thiophényle), du propanoate d'éthyl-3-oxo-3-(3-thiophényle), du propanoate de méthyl-3-oxo-3-(2-furanyle), du propanoate d'éthyl-3-oxo-3-(2-furanyle), du propanoate de méthyl-3-oxo-3-(3-furanyle), du propanoate d'éthyl-3-oxo-3-(3-furanyle), du propanoate de méthyl-3-oxo-3-(2-pyridinyle), du propanoate d'éthyl-3-oxo-3-(2-pyridinyle), du propanoate de méthyl-3-oxo-3-(3-pyridinyle), du propanoate d'éthyl-3-oxo-3-(3-pyridinyle), du propanoate de méthyl-3-oxo-3-(4-pyridinyle), du propanoate d'éthyl-3-oxo-3-(4-pyridinyle) ou du N-méthylamide de l'acide de 3-oxo-3-(2-thiophényl)propanoïque sont utilisés en tant que composés de formule (I).

4. Procédé selon au moins l'une quelconque des revendications 1 à 3, **caractérisé en ce que** la somme des concentrations des composés de formules (I) et (II) dans la suspension cellulaire est comprise entre 1 et 900 mM.

5. Procédé selon au moins l'une quelconque des revendications 1 à 4, **caractérisé en ce que** le procédé est réalisé en présence d'un solvant organique.

6. Procédé selon au moins l'une quelconque des revendications 1 à 5, **caractérisé en ce que** du propanoate de (S)-méthyl-3-hydroxy-3-(2-thiophényle), du propanoate de (S)-éthyl-3-hydroxy-3-(2-thiophényle), du propanoate de (S)-méthyl-3-hydroxy-3-(3-thiophényle), du propanoate de (S)-éthyl-3-hydroxy-3-(3-thiophényle), du propanoate de (S)-méthyl-3-hydroxy-3-(2-furanyle), du propanoate de (S)-éthyl-3-hydroxy-3-(2-furanyle), du propanoate de (S)-méthyl-3-hydroxy-3-(3-furanyle), du propanoate de (S)-éthyl-3-hydroxy-3-(3-furanyle), du propanoate de (S)-méthyl-3-hydroxy-3-(2-pyridinyle), du propanoate de (S)-éthyl-3-hydroxy-3-(2-pyridinyle), du propanoate de (S)-méthyl-3-hydroxy-3-(3-pyridinyle), du propanoate de (S)-éthyl-3-hydroxy-3-(3-pyridinyle), du propanoate de (S)-méthyl-3-hydroxy-3-(4-pyridinyle), du propanoate de (S)-éthyl-3-hydroxy-3-(4-pyridinyle) ou du N-méthylamide de l'acide (S)-3-hydroxy-3-(2-thiophényl)-propanoïque sont préparés en tant que composés de formule (II).

7. Procédé selon au moins l'une quelconque des revendications 1 à 6, **caractérisé en ce que** R² et R³ représentent chacun indépendamment l'un de l'autre hydrogène, méthyle, éthyle, isopropyle, phényle ou benzyle dans les formules (III), (IV) et (VI).

8. Procédé selon au moins l'une quelconque des revendications 1 à 7, **caractérisé en ce que** des composés de formule (I) sont obtenus par mise en réaction de composés de formule (VII)
hétéroaryle-CO-CH₃ (VII)
dans laquelle hétéroaryle a la signification et les plages préférentielles indiquées pour la formule (VI)
avec des composés de formule (VIII)
R¹-O-W (VIII)
dans laquelle
R¹ et W ont les mêmes significations que celles indiquées pour la formule (I), en présence d'une base.

9. Procédé selon une ou plusieurs des revendications 1 à 8, **caractérisé en ce que** la réduction de composés de formule (IV) a lieu avec des hydrures de bore ou d'aluminium complexes.

10. Procédé selon au moins l'une quelconque des revendications 1 à 9, **caractérisé en ce que** du (1S)-3-(méthylamino)-1-(2-thiophényl)-1-propanol, du (1R)-3-(méthylamino)-1-(2-thiophényl)-1-propanol, du (1S)-3-(diméthylamino)-1-(2-thiophényl)-1-propanol ou du (1R)-3-(diméthylamino)-1-(2-thiophényl)-1-propanol est préparé.

11. Procédé selon au moins l'une quelconque des revendications 1 à 10, **caractérisé en ce que**
- lors d'une étape d),
les composés de formule (VI) enrichis énantiomériquement sont mis en réaction en présence d'une base avec des composés de formule (XI)
R⁶-Hal (XI)
dans laquelle
R⁶ représente phényle ou naphtyle, qui peut ne pas être substitué ou être substitué une ou plusieurs fois par des substituants choisis à chaque fois indépendamment les uns des autres dans le groupe constitué par cyano, CO-(alkyle en C₁-C₁₂), O-(alkyle en C₁-C₁₂), (alkyle en C₁-C₁₂), fluor, chlore, brome, fluoroalkyle en C₁-C₁₂, et
Hal représente fluor, chlore, brome ou iode,
pour former des composés de formule (X) enrichis énantiomériquement
hétéroaryle-CH(OR⁶)-CH₂-CH₂NR₂R³ (X)
dans laquelle hétéroaryle, R² et R³ ont la signification indiquée pour la formule (VI) et R⁶ a la signification indiquée pour la formule (XI).

12. Procédé selon la revendication 11, **caractérisé en ce que** de la (S)-N-méthyl-3-(1-naphtalényloxy)-3-(2-thiényl)propylamine et de la (R)-N-méthyl-3-(1-naphtalényloxy)-3-(2-thiényl)propylamine ou leurs sels d'ammonium sont préparés.
